# EUROPEAN PATENT APPLICATION

(11) **EP 0 546 718 A2**
(43) Date of publication of application: **16.06.1993**
(21) Application number: 92310771.8
(22) Date of filing: 25.11.1992
(51) Int. Cl.: C07C 43/188, C07C 321/28, C07C 41/01, C07C 319/14, C08K 5/00

(54) **Synthesis of derivatized fullerenes**

(30) Priority: 27.11.1991 US 800789; 04.12.1991 US 803003
(71) Applicant: EXXON RESEARCH AND ENGINEERING COMPANY, Florham Park, New Jersey 07932-0390 (US)
(72) Inventor: Miller, Glen Paul, Easton, Pennsylvania (US); Hsu, Chang Samuel, Bridgewater, New Jersey (US); Millar, John Manuel, Pittstown, New Jersey (US)
(74) Representative: Fletcher Watts, Susan J.

(57) **Abstract**

This invention relates to novel compositions of matter, certain derivatized fullerenes, and the process of making them. The derivatized fullerenes of the present invention contain substituent groups in even numbers and are produced by contacting the fullerenes with an oxidizing agent, such as a strong acid or superacid, then adding a suitable nucleophilic quenching agent such as an alcohol, thiol or aromatic that contains the substituent group to be added. Terminal 1,y-difulleroxyalkanes result from trapping with terminal 1,y-diols where y is the number of carbon atoms in the diol. Odd numbered derivatized fullerenes are produced by heating the even number derivatized fullerenes thus produced. The compositions may be used as crosslinking agents in polymers, core building blocks for star polymers, and stiffening agents in polymers and polymer blends.

## Description

This invention relates to derivatized fullerene compounds and their method of preparation.

The present invention provides fullerenes derivatized by addition of nucleophilic groups (from suitable nucleophiles), and the process of making these compositions from fullerenes using oxidizing agents such as strong acids and/or superacids, and suitable nucleophiles such as alcohols, thiols, aromatics and terminal 1,y-diols to form the corresponding nucleophilic addition products. The invention also includes the products produced by the process disclosed herein. As used herein, the term terminal 1,y-diol means diols wherein y denotes the number of carbon atoms in the diol molecule, and is an integer greater than or equal to 2. The upper number for y is limited simply by the current state of the art, i.e. it is the largest chain length available. The extent of nucleophilic addition in the fullerene derivatives is determined by the nature of the starting fullerene material, the strength of the oxidizing agent used, and the nature of the nucleophile used. In general, stronger oxidizing agents may be expected to lead to a greater level of nucleophilic addition (i.e., add a larger number of nucleophilic groups to the fullerenes).

The derivatized fullerenes of the present invention may be used as stiffening agents in polymers and/or polymer blends or as crosslinking agents in polymers and/or core building blocks for star polymers.

Fullerenes may be purchased from commercial sources or may also be synthesized by graphite volatilization, as descried by W. Krätschmer, et al., Nature, 347 (1990), and extracted from the resulting soot by toluene extraction, as described by D. M. Cox, et al., 113 J. Am. Chem. Soc., 2940 (1991). The resulting material generally contains from about 70% to about 85% C₆₀ and lesser amounts of higher fullerenes. All other materials described herein may be obtained from commercial sources and were used, as obtained, without further purification, unless otherwise specified.

The fullerenes starting material contains either pure C₆₀ or a mixture of C₆₀ and higher fullerenes. However, to the extent that samples containing only one type of higher fullerene are available, they are equally acceptable starting materials for the process of the present invention.

In the process of the present invention, the fullerenes are derivatized by adding nucleophilic groups to form the corresponding nucleophilic addition products by first contacting the fullerenes with an oxidizing agent, resulting in the formation of fullerene radical cation, followed by nucleophilic trapping (i.e., quenching) of that cation using a suitable nucleophile.

The oxidizing agent may be an acid, the nature of which will influence the degree of nucleophilic addition to the fullerene. Thus, weaker oxidizing agents tend to support a lesser degree of nucleophilic addition to a given fullerene than do stronger oxidizing agents. However, it is generally expected that two or other even numbers of additions will occur, typically 2, 4, or 6 additions. The addition of nucleophilic groups may occur symmetrically, although this is not required. The oxidizing agent may be a Lewis acid, preferably selected from the group consisting of SbCl₅, SbF₅, SbBr₅, TaCl₅, TaF₅, TaBr₅, NbCL₅, NbF₅, NbBr₅, AsCl₅, AsF₅, AsBr₅, PCl₅, PBr₅ and SO₃ or a Bronsted acid, preferably selected from the group consisting of H₂SO₄ (all weight percents with water), CF₃SO₃H, CH₃SO₃H, FSO₃H, ClSO₃H and HF; or a Bronsted-Lewis acid composite, preferably selected from the group consisting of fuming sulfuric acid (i.e., mixtures of H₂SO₄ and free SO₃ in all proportions), FSO₃H-SbF₅, HF-SbF₅, CF₃SO₃H-TaF₅, HF-TaF₅ and FSO₃H-TaF₅.

The fullerene radical cation generated in oxidizing media is quenched (by nucleophilic trapping) with an excess of a suitable nucleophile. Thus, for example, if it is desired that the process produce a derivatized fullerene containing methoxy groups as the nucleophilic groups, methanol is employed as the nucleophile (quenching agent); if ethoxy groups, ethanol should be used; if phenyl groups, benzene should be used; if it is desired to produce an alkyl fullerene sulfide, alkyl thiols should be used; if an aryl fullerene sulfide, thiophenol or other aryl thiols should be used; if the derivatized fullerene is to be a 1,y-difulleroxyalkane, the corresponding terminal 1,y-diols should be used. The quenching is best performed at low temperature, preferably below room temperature. If the nucleophile (i.e., quenching agent) has a low melting point, it may be pre-chilled by any of numerous procedures known to one skilled in the art, including placing the container holding the nucleophile into a cryogenic bath or pouring liquid nitrogen into the container holding the nucleophile in order to generate a liquid nitrogen/nucleophilic slurry. If nucleophiles possessing higher melting points are employed as quenching agents, it is desirable to add a non-nucleophilic solvent that will act as a freezing point depressant. Such a freezing point depressant should be miscible with both oxidizing agent and nucleophile. For example, sulfur dioxide (SO₂), sulfuryl fluoride chloride (SO₂FCl), nitromethane (CH₃NO₂), chloroform (CHCl₃) or a freon may be employed in this manner.

After quenching the oxidized fullerene with the nucleophile, the resulting derivatized fullerene can be either extracted with appropriate solvent (e.g., methylene chloride, benzene, toluene), or separated from the reaction mixture by other convenient means (e.g., centrifuge, column chromatography, prep scale high pressure liquid chromatography), which are readily known to one of ordinary skill in the art.

The time required to carry out the process of the present invention will vary, depending upon the particular reaction conditions and difficulty in separating the derivatized fullerene from the reaction mixture. For example, the length of time that the fullerene is allowed to contact the oxidizing agent may be variable. The actual quench of the fullerene radical cation generally takes less than a few minutes. Chilling the nucleophile and/or nucleophile/non-nucleophilic solvent mixture to the appropriate temperature may require more time, depending upon the volume of solution involved. Separation of derivatized fullerene can take from about a few minutes to several hours, depending upon the ease of separation and the separation method used.

The process produces derivatized fullerenes (i.e., fullerenes having nucleophilic groups) having the general formula Cₓ(Nu)ₙ, wherein Cₓ is a fullerene, x being an even number greater than 30, Nu is a nucleophilic group, and n is an even number greater than or equal to 2. The fullerene, Cₓ, is preferably C₆₀ or a higher fullerene or a mixture of one or more fullerenes. The maximum number for x on the maximum size possible for a fullerene. According to the current state of the art this is believed to be about 420, although this may increase in the future. Thus, for the derivatized fullerenes produced from nucleophilic trapping with an alcohol, the nucleophilic group, Nu, has the general formula OR, wherein O is oxygen and R is an organic moiety such as -CH₃, -C₂H₅ and -C₄H₉, and wherein n is an even number. For the derivatized fullerenes resulting from nucleophilic trapping with either thiols or aromatics (i.e., fullerene sulfides and arylated fullerenes, respectively), the nucleophilic group, Nu, has the formula of the corresponding thiol or aromatic, respectively, and n is an even number. All other terms are as defined in the general formula. The even number nucleophilic addition products (derivatized fullerenes) fragment upon heating into odd numbered nucleophilic addition products (derivatized fullerenes).

For the derivatized fullerenes resulting from nucleophilic trapping with terminal 1,y-diols, the process produces 1,y-difulleroxyalkanes of the general formula Cₓ(O-(CH₂)ₙ-O)Cₓ where Cₓ is a fullerene, preferably C₆₀, higher fullerenes, and mixtures thereof, wherein n is equal to y, and wherein y is the number of carbon atoms in the terminal 1,y-diol molecule. However, as indicated by the foregoing formula, two fullerene molecules are required for every one diol molecule in order to produce the difulleroxyalkanes of the present invention.

Another embodiment of the present invention concerns novel compositions of matter comprising derivatized fullerenes (i.e., fullerenes having nucleophilic groups as descried herein) and includes the products produced by the process of the present invention. The novel compositions of the present invention are prepared from samples containing C₆₀ and from mixtures of C₆₀ and higher fullerenes, but may also be prepared from starting materials containing any one given type of fullerene. Preparation of the starting materials is described above. The relative proportion of each type of derivatized fullerene will depend on the identity and proportion of the fullerene(s) contained in the starting material. The resulting novel compositions will contain two or more even numbers of nucleophilic groups. The number and type of nucleophilic groups on the derivatized fullerene are influenced by the size of the starting fullerene, as well as the nature of the oxidizing medium and the the quenching medium, respectively, that are used to synthesize the derivatized fullerene. Thus, for example where the fullerene is C₆₀ and the oxidizing medium is 100% Magic Acid (1:1 FSO₃H-SbF₅), and the nucleophile is an alcohol, the novel compositions will include alkoxylated C_{60'}s selected from C₆₀(OR)₂, C₆₀(OR)₄, and C₆₀(OR)₆, wherein O is oxygen and R is an organic moiety containing 1 to 22 carbon atoms, more preferably 1 to 10 carbon atoms, and most preferably is -CH₃, -C₂H₅, and -C₄H₉. Upon heating, these compounds fragment to odd numbered alkoxy-substituted fullerenes. Similarly, where the fullerene is C₆₀, the oxidizing medium is 100% Magic Acid (1:1 FSO₃H-SbF₅) and the trapping nucleophile is either a thiol or an aromatic, the novel compositions will include C₆₀(Nu)₂, C₆₀(Nu)₄, and C₆₀(Nu)₆, wherein Nu results from addition of the appropriate thiol or aromatic nucleophile, such as thiophenol or benzene, respectively, to the C₆₀ radical cation (i.e., oxidized C₆₀). Upon heating, these compounds fragment into odd numbered nucleophilic addition products. The compounds of the present invention are made using the process as discussed above.

The present invention will be further understood bY reference to the following examples, which are intended to demonstrate the invention and not limit it in any way.

### Example 1

29 mg of a mixture of fullerenes containing approximately 80-85 wt.% C₆₀ prepared according to the process described in Kratschmer, Lamb, Fostiropoulos and Huffman, Nature 347, 354-58 (1990) and in Cox, et al, J.Am.Chem.Soc. 113, 2940-44 (1991), was added to a mixture of 1.0 ml of 1:1 FSO₃H-SbF₅ as the oxidizing agent and 0.28 ml of a non-nucleophilic solvent, SO₂FCl, at -80°C. The resulting reaction mixture was sealed in a glass vessel and then allowed to warm to 25°C for approximately 48 hours. The sealed glass reaction vessel was then frozen in liquid nitrogen and opened. As the reaction mixture thawed, it was added dropwise to a cryogenic mixture of methanol and liquid nitrogen at approximately -100°C. Separation of the methoxylated fullerenes thus formed was carried out by first neutralizing the reaction mixture with aqueous sodium hydroxide, followed by extraction with methylene chloride three times. The combined methylene chloride layers from the extractions were evaporated at reduced pressure (10 mm Hg), leaving a dark solid behind. The solid was identified as methoxylated fullerenes. Characterization of the products by ¹³C NMR revealed the presence of 3 unique methoxylated fullerenes, each with a symmetrical structure, the di, tetra, and hexamethoxylated C₆₀ (i.e., C₆₀(OCH₃)₂, C₆₀(OCH₃)₄, and C₆₀ (OCH₃)₆). Negative ion chemical ionization mass spectrometry revealed the addition of 1, 2, 3, 4, 5 and 6 methoxy units to C₆₀. The presence of signals corresponding to methoxylated fullerenes containing 1, 3, and 5 methoxy units in the mass spectrum is attributable to fragmentation of the symmetrical methoxylated fullerenes of the formulas C₆₀(OCH₃)₂, C₆₀(OCH₃)₄, and C₆₀(OCH₃)₆ during ionization of the sample. The concentration of higher fullerenes in the original sample was too small to permit the detection of their methoxylated derivatives by ¹³C NMR spectroscopy or mass spectrometry.

### Example 2

Using 29 mg of pure C₆₀, the process of Example 1 was repeated except that isolation of solid methoxylated C₆₀ was accomplished by centrifuge.

### Example 3

Using 20 mg of pure C₆₀, the process of Example 2 was repeated except that the alcohol employed as nucleophilic trap was 1-butanol present as a mixture with nitromethane. The 1-butanol/nitromethane mixture was kept at approximately -10°C by placing the vessel holding the mixture in a cryogenic bath. The solid butoxylated C₆₀ products were isolated by centrifuge. NICI-MS spectra revealed the addition of 1 and 2 butoxy groups to C₆₀, the monoaddition product being the result of fragmentation of dibutoxy C₆₀. Evidence for higher addition products also exists in the NICI-MS spectra, although the corresponding signals are relatively weak.

### Example 4

Using 20 mg of pure C₆₀, the process of Example 1 was repeated, except that the nucleophile employed was thiophenol. NICI-MS analysis revealed the addition of 1, 2, 3, 4, 5 and 6 -C₆H₅S units to C₆₀, the 1, 3 and 5 addition products resulting from fragmentation in the mass spectrometer.

### Example 5

The process of Example 1 was repeated, except that the nucleophile was 1,6-hexanediol. NICI-MS analysis of the reaction products revealed a large signal corresponding to 1,6-difulleroxhexane (MW = 1556 amu's) which may be a stable compound, a fragmentation product of a higher molecular weight derivative, or both.

## Claims

1. A process for preparing a derivatized fullerene, comprising:
(a) contacting a fullerene or mixture of fullerenes with an oxidizing agent; then
(b) adding a nucleophile selected from aromatics, thiols, and alcohols to the fullerene-oxidizing agent mixture in order to produce a derivatized fullerene having the formula Cₓ(Nu)ₙ, wherein n is an even number from 2 to 6, Nu is a nucleophilic group from the corresponding nucleophile, and Cₓ is a fullerene.

2. The process of claim 1 further comprising heating the derivatized fullerenes in order to produce derivatized fullerenes having an odd number of nucleophilic groups.

3. The process of claim 1 wherein the alcohol is selected from methanol, ethanol, and 1-butanol.

4. A process for preparing a derivatized fullerene, comprising:
(a) contacting a fullerene or mixtures of fullerenes with an oxidizing agent; then
(b) adding a terminal 1,y-diol, wherein y is the number of carbon atoms in the diol molecule, to the fullerene-oxidizing agent mixture for a time sufficient to produce a derivatized fullerene having the formula Cₓ(O-(CH₂)ₙ-O)Cₓ where n is equal to y and Cₓ is a fullerene.

5. The process of any preceding claim wherein the oxidizing agent is a Bronsted acid selected from H₂SO₄, CF₃SO₃H, CH₃SO₃H, FSO₃H, CF₃SO₃H and HF; or a Lewis-Acid selected from SbCl₅, SbF₅, TaCl₅, TaF₅, TaBr₅, NbCl₅, NbF₅, NbBr₅, AsCl₅, SbBr₅, AsF₅, PCl₅, PBr₅ and SO₃; or a Bronsted-Lewis acid composite selected from fuming sulfuric acid, FSO₃H-SbF₅, HF-SbF₅, CF₃SO₃H-TaF₅, HF-TaF₅ and FSO₃H-TaF₅.

6. A composition comprising a derivatized fullerene having the formula Cₓ(Nu)ₙ wherein Nu is a nucleophilic group that results from trapping of oxidized Cₓ with nucleophiles, Cₓ is a fullerene, and n is an even number from 2 to 6.

7. The composition of claim 6 wherein the nucleophilic group is selected from -OC₂H₅, -OC4H₉ and C₆H₅S.

8. The composition of claim 6 or 7 wherein the derivatized fullerene having the formula Cₓ(Nu)ₙ is selected from C₆₀(Nu)₂, C₆₀(Nu)₄, and C₆₀(Nu)₆, and mixtures thereof.

9. A composition comprising terminal 1,y-difulleroxyalkanes produced by addition of a terminal 1,y-diol to two molecules of fullerenes, wherein y is the number of carbon atoms in the diol and wherein said difulleroxyalkane has the formula Cₓ(O-(CH₂)ₙ-O)Cₓ wherein n is equal to y and wherein Cₓ is a fullerene.

10. A composition comprising a derivatized fullerene having nucleophilic groups that result from trapping of oxidized fullerene with one or more nucleophiles selected from alcohols, thiols and aromatics, wherein said groups are present in odd numbers.
